(19)

Europäisches
Patentamt
European
Patent Office
Office européen
des brevets

(11) **EP 3 858 974 A1**

(12) **EUROPEAN PATENT APPLICATION**
published in accordance with Art. 153(4) EPC

(43) Date of publication:
**04.08.2021 Bulletin 2021/31**

(21) Application number: **19867121.6**

(22) Date of filing: **11.09.2019**

(51) Int Cl.:
*C12M 3/00* *(2006.01)*    *B81B 1/00* *(2006.01)*
*C12M 1/00* *(2006.01)*

(86) International application number:
**PCT/JP2019/035653**

(87) International publication number:
**WO 2020/066609 (02.04.2020 Gazette 2020/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.09.2018 JP 2018185218**

(71) Applicant: **Ushio Denki Kabushiki Kaisha
Tokyo 100-8150 (JP)**

(72) Inventor: **YAMANAKA,Makoto
Tokyo 100-8150 (JP)**

(74) Representative: **Tomerius, Isabel
Lang & Tomerius
Patentanwaltspartnerschaft mbB
Rosa-Bavarese-Strasse 5
80639 München (DE)**

(54) **CELL CULTURE CHIP**

(57)    A cell culture chip from which a liquid retained in an opening is able to be sucked by a simple operation procedure while a liquid in a channel remains is provided.

A cell culture chip according to the present invention includes a bottom portion; a base portion formed on an upper surface of the bottom portion; a first well provided by opening the base portion in a first direction extending from a portion of a main surface of the base portion toward the bottom portion and having a shape such that a capillary force thereof is smaller than that of the chamber; a second well provided by opening the base portion in the first direction at a position separated from the first well in a second direction parallel to the main surface; and a tubular chamber that is defined by a region sandwiched between the bottom portion and the base portion and that provides communication between the first and second wells in the second direction. The first well has a shape with which a capillary force of the first well is smaller than a capillary force of the chamber.

FIG. 2

## Description

Technical Field

The present invention relates to a cell culture chip.

Background Art

Cells exist, in a living body and tissue, in an "extracellular microenvironment" including (i) soluble factors, such as growth factors, vitamins, and gas molecules, (ii) insoluble factors, such as extracellular matrix proteins, rigidity, and pressure, and (iii) cell-cell interactions. The function of cells is controlled while these factors are complexly and strictly controlled. That is, in order to freely control the function of target cells such as human pluripotent stem cells (human ES/iPS cells), which are promising for regenerative medicine, cell transplantation therapy, drug development, and so forth, it is essential to freely control the extracellular microenvironment.

Conventionally, culture and experiments of cells containing human ES/iPS cells have been performed under a two-dimensional environment using culture dishes or plates. However, it is considered that cells are originally placed under a three-dimensional environment and the original function cannot be expressed under a two-dimensional environment. Also in tissue engineering using human ES/iPS cells, it is very important to prepare a three-dimensional environment.

The size of the extracellular microenvironment is also a very important factor. Cells are controlled in a living body under a micrometric ($\mu$m) scale microenvironment. However, in the conventional culture method, it has been difficult to control factors in such a micro space. Furthermore, it has been almost impossible to exhaustively analyze these factors. Under such circumstances, a technique for creating a three-dimensional cell culture environment, which has been difficult in the conventional method, has been desired.

As means for realizing such a three-dimensional cell culture environment, a microchannel chip disclosed in the following PTL 1 has been proposed.

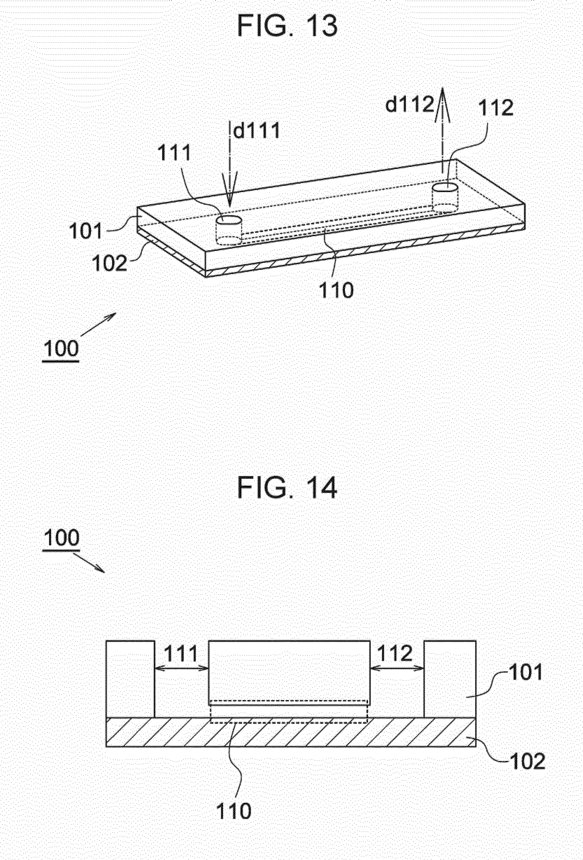

Fig. 13 is a perspective view schematically illustrating a structure of a microchannel chip disclosed in the following PTL 1. A microchannel chip 100 includes a base 101 and a resin film 102, openings are formed at two positions on a main surface side of the base 101, and these openings constitute an inflow port 111 and an outflow port 112. Also, a channel groove 110 is formed to communicate with these openings (111, 112). The groove 110 is covered with the resin film 102 and constitutes a tubular channel. Fig. 14 is a sectional view schematically illustrating the structure of the microchannel chip 100.

At the time of inspection, a target liquid sample is poured from the inflow port 111 in a direction d111. The liquid sample flows in the groove 110 toward the outflow port 112. A portion of the groove 110 also serves as a detection portion. For example, a substance that emits fluorescence by reaction with a detection target substance, such as a specific protein, is immobilized in the middle of the groove 110. The portion (the detection portion) is observed using a fluorescence microscope, and hence it is determined whether or not the liquid sample includes the specific detection target substance.

Citation List

Patent Literature

PTL 1: Japanese Unexamined Patent Application Publication No. 2018-047614

Summary of Invention

Technical Problem

A microchannel chip is used for culturing cells in a culture space under a predetermined environment and observing the state of the cells. For the culture, a liquid culture solution is used as a culture medium.

When cells are cultured under a specific environment for a constant period of time (days) or longer, an operation of exchanging a culture medium (a culture solution) may be required for the purpose of maintaining a culture environment, supplying nutrients to the cells, removing waste products, and so forth. For example, in the case where cells are cultured using the microchannel chip 100 illustrated in Fig. 13, when it is necessary to exchange a culture solution, an operation of removing the culture solution from the inflow port 111 or the outflow port 112 and replenishing (adding) a new culture solution is performed. Specifically, the culture solution is removed by performing a vacuum operation from the inflow port 111 or the outflow port 112 using a pipet of which the inside is decompressed. Hereinafter, a case where an operation of extracting a culture solution from the inflow port 111 side using a pipet is performed will be described, however the description is similar to that of a case where a culture solution is extracted from the outflow port 112 side.

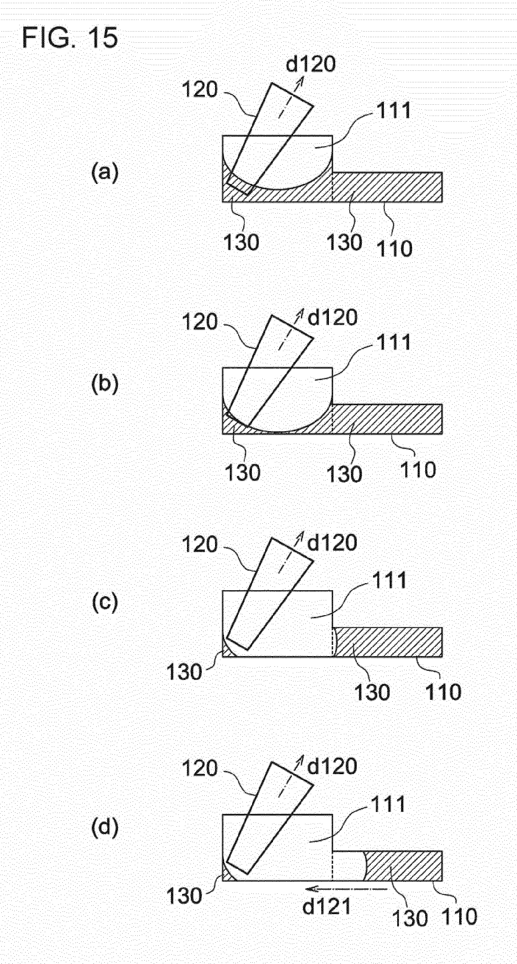

When a suction operation of a culture solution by a pipet is performed in a state in which the tip of the pipet is located on the inflow port 111 side, not only the culture solution in the inflow port 111 but also the culture solution in the groove 110 are sucked unless the suction force is strictly adjusted. This point will be described in detail with reference to Fig. 15.

Fig. 15 is a view schematically illustrating a change in the remaining amount of a culture solution when the culture solution is sucked from the conventional microchannel chip 100 described above with reference to Fig. 13 and Fig. 14. Fig. 15 schematically illustrates how the remaining amount of the culture solution changes (decreases) in the order of (a), (b), (c), and (d) over time. Also, referring to Fig. 15, a region in which a culture solution 130 exists is hatched.

When the culture solution is sucked from the conventional microchannel chip 100, the culture solution 130 is sucked in a d120 direction in a state in which the tip of a pipet 120 is inserted into the inflow port 111. More specifically, the culture solution 130 is sucked while the tip of the pipet 120 is pressed against a bottom surface or a side wall in the vicinity of the bottom surface of the inflow port 111. As the suction of the culture solution 130 progresses, the liquid level of the culture solution 130 is gradually lowered (see Fig. 15(a) and Fig. 15(b)).

When the suction of the culture solution 130 further progresses, as illustrated in Fig. 15(c), the bottom surface of the inflow port 111 is exposed. At this time point, as illustrated in Fig. 15(c), the culture solution 130 slightly remains in a corner portion of the inflow port 111.

When the suction of the culture solution 130 further progresses from the state of Fig. 15(c), as illustrated in Fig. 15(d), the culture solution 130 existing in the groove 110 constituting the channel is guided to the inflow port 111 side (a d121 direction) along an inner wall of the groove 110 while the culture solution 130 remains in the corner portion of the inflow port 111, and then sucked by the pipet 120. Consequently, as illustrated in Fig. 15(d), a portion of the culture solution 130 in the groove 110 is sucked by the pipet 120.

As described above, the culture solution 130 is sucked, for example, to exchange the culture solution 130. That is, after the culture solution 130 retained on the inflow port 111 side is removed, for example, as illustrated in Fig. 15(d), a new culture solution (hereinafter, referred to as "culture solution 130a" for the convenience of description) is poured from the inflow port 111 side. The new culture solution 130a flows into the groove 110 through the inflow port 111. Consequently, the old culture solution 130 already existing in the groove 110 is pushed out to the outflow port 112 side. Thus, the culture solution 130 in the groove 110 is exchanged for the new culture solution 130a.

However, as illustrated in Fig. 15(d), in a state in which the portion of the culture solution 130 in the groove 110 has been sucked when the new culture solution 130a is made to flow in, an air bubble may remain in a boundary region between the new culture solution 130a and the existing culture solution 130 in the groove 110. When the new culture solution 130a is gradually made to flow in while the air bubble exists, the air bubble moves to be pushed out in the groove 110 by the culture solution 130a. At this time, in the groove 110, more specifically, cells cultured in a state of being attached to a bottom surface of the groove 110 may be separated by a force generated at the interface of the air bubble and may flow together with the culture solution (130/130a).

Also, even when the cells can be retained in the groove 110 during execution of the exchange operation of the culture solution, the exchange operation of the culture solution may be completed in the state in which the air bubble remains in the groove 110. In this case, since the volume occupied by the air bubble and the surface of the culture chamber are no longer used for the culture, the original total number of cells and the original total amount of culture solution are randomly decreased, and thus an accurate cell test is no longer performed. Also, the intended flow and diffusion of the culture solution designed through the channel shape are hindered, and there is a possibility that cells are no longer cultured under a target environment. For example, there are possibilities that the concentration of a culture solution component to be transported to the cells is disrupted, shear stress applied to the cells is changed, and waste products or residues generated from the cells remain.

For example, physiologically active substances (for example, cytokines, hormones, lipids, extracellular matrices, microRNAs, exosomes, nutrients, or drugs that exhibit endocrine functions) are released from cells. When the physiologically active substances collide with the wall surface covering the groove 110 and are returned toward the cells, the physiologically active substances may act on the cells. However, when an air bubble is formed in the groove 110, the flow of the physiologically active substances is hindered, and the culture state of the cells may be affected.

In view of the above-described circumstances, when the culture solution 130 is exchanged, it is necessary to adjust the suction force not to suck the culture solution 130 in the groove 110. However, in the conventional microchannel chip 100, the opening (the inflow port 111/the outflow port 112) has a typical tubular shape (cylindrical shape), and no consideration is made to adjust the suction force of the culture solution 130. Thus, when the suction operation of the culture solution 130 is performed, it is necessary to perform strict adjustment such that the suction operation is suspended immediately before the suction of the culture solution 130 existing in the groove 110 is started while the culture solution 130 retained in the opening (the inflow port 111/the outflow port 112) is removed.

Thus, when the suction operation is performed, an operator is required to have strict adjustment skill, and there is a problem of poor reproducibility. Also, the existence of such a situation becomes an obstacle to automation of the suction operation.

In view of the above-described problems, it is an object of the present invention to provide a cell culture chip from which a liquid retained in an opening is able to be sucked by a simple operation procedure while a liquid in a channel remains.

Solution to the Problem

A cell culture chip according to the present invention includes

a bottom portion;
a base portion formed on an upper surface of the bottom portion;
a first well provided by opening the base portion in a first direction extending from a portion of a main surface of the base portion toward the bottom portion, the main surface being a surface opposite to the bottom portion;
a second well provided by opening the base portion in the first direction at a position separated from the first well in a second direction parallel to the main surface; and
a tubular chamber that is defined by a region sandwiched between the bottom portion and the base portion and that provides communication between the first well and the second well in the second direction.

The first well has a shape such that a capillary force of the first well is smaller than a capillary force of the chamber.
In the specification, the term "a capillary force of a well" refers to a capillary force generated in a corner portion where an inner surface (an inner wall) of the well and a bottom surface (an inner bottom wall) of the well intersect with each other.
As described above with reference to Fig. 15, when the suction operation by the pipet 120 is continued in a state in which the conventional microchannel chip 100 is filled with the culture solution 130, the suction of the culture solution 130 retained in the channel (the groove 110) is started even though a portion of the culture solution 130 remains in the opening (the inflow port 111). As illustrated in Fig. 15(c), it is considered that this is because the capillary force of the liquid pool portion of the culture solution 130 formed in the corner portion of the inflow port 111 is larger than the capillary force of the groove 110.
Both the inner wall of the groove 110 and the bottom surface of the opening (the inflow port 111) are highly hydrophilic, and the surfaces thereof are in a slightly wet state. Thus, when the suction operation is continued from the state of Fig. 15(c), the culture solution 130 retained in the groove 110 having a capillary force smaller than that of the corner portion of the inflow port 111 moves along the inner wall of the groove 110 and the bottom surface of the inflow port 111 to the corner portion side of the inflow port 111 having a large capillary force. Consequently, as illustrated in Fig. 15(d), it is considered that the culture solution 130 retained in the corner portion of the inflow port 111 is hardly sucked and the suction of the culture solution 130 in the groove 110 is continued.
In contrast, with the cell culture chip according to the present invention, the first well has the shape such that the capillary force of the first well is smaller than the capillary force of the chamber. Consequently, when the suction is started from the first well side, the suction of the liquid retained in the chamber is not started until the suction of the liquid (the culture solution) retained in the first well is substantially completed. Thus, the operator only has to perform the suction of the liquid retained in the first well with a suction force to the extent that the liquid retained in the first well can be sucked and to stop the suction operation at the time point when the suction from the first well is completed, and it is not necessary to strictly adjust the suction force or the suction period of time in the suction operation.
Consequently, the operation of the operator is simplified compared to the operation of exchanging the culture solution for the conventional chip, and no special skill is required, thereby improving workability. Also, since it is only necessary to suck the liquid retained in the first well with the suction force to the extent that the liquid retained in the first well can be sucked, and strict adjustment is no longer necessary, it is possible to automate the suction operation.
In the cell culture chip, the bottom portion and the base portion may be integrally made of the same material, or may be made of different materials.
The chamber may be a chamber (a culture chamber) constituting a culture space. Also, the chamber may include the culture chamber and a communication channel that provides communication between the culture chamber and the first well in the second direction. In the latter case, the capillary force of the first well may be smaller than the capillary force of the communication channel constituting the chamber.
In the cell culture chip, the first well may have a reduced-diameter region of which an opening diameter continuously decreases without increasing toward the bottom portion at a position closer to the bottom portion than the main surface of the base portion.
With the above-described configuration, the first well has a shape of which an opening diameter at a position close to the chamber is smaller than an opening diameter at a position far from the chamber. With the shape, the capillary force at the position of the bottom surface of the first well can be reduced.

The first well may have an inner wall defined by the base portion, and
the inner wall may include a curved surface or a flat surface non-parallel to the main surface in the reduced-diameter region of the first well.

With the configuration, in the first well, an inclined surface is formed at the corner portion in the vicinity of the bottom surface, and the corner angle of the corner portion between the inclined surface and the bottom surface of the first well is an obtuse angle. Thus, the capillary force of the corner portion of the first well is reduced.

The cell culture chip may include a communication well that is formed continuously with the first well in the first direction and that provides communication between the reduced-diameter region of the first well and the chamber in the first direction, and
the communication well may have a bottom surface defined by the bottom portion and may have an opening diameter smaller than the opening diameter of the reduced-diameter region of the first well.

As described above, since the first well has the reduced-diameter region, the first well has a shape of which the opening diameter decreases toward the bottom portion at a position close to the bottom portion. Thus, the thickness of the base portion is small at the position at which the opening diameter is the smallest, and molding may become difficult.

In contrast, by providing the communication well like the above-described configuration, the thickness of the base portion located around the communication well is ensured and hence molding is facilitated. Also, since the opening diameter of the communication well is smaller than that of the reduced-diameter region of the first well, the communication well does not hinder the suction of only the liquid retained in the first well at the time of sucking the liquid. That is, even when a portion of the liquid remains in the communication well, the liquid retained in the first well can be substantially completely sucked. In other words, even with the configuration provided with the communication well, it is possible to substantially completely suck the liquid retained in the first well without sucking the liquid retained in the chamber.

In the cell culture chip, a bottom surface of the first well may be defined by the bottom portion.

Also, in the cell culture chip, the second well may have a shape such that a capillary force of the second well is smaller than a capillary force of the chamber. In this case, the liquid can be sucked from either one of the first well and the second well while the liquid is retained in the chamber.

The second well may have a reduced-diameter region of which an opening diameter continuously decreases without increasing toward the bottom portion at a position closer to the bottom portion than the main surface of the base portion.

Also, a cell culture chip according to the present invention includes

a bottom portion;
a base portion formed on an upper surface of the bottom portion;a first well provided by opening the base portion in a first direction extending from a portion of a main surface of the base portion toward the bottom portion, the main surface being a surface opposite to the bottom portion;
a second well provided by opening the base portion in the first direction at a position separated from the first well in a second direction parallel to the main surface; and a tubular chamber that is defined by a region sandwiched between the bottom portion and the base portion and that provides communication between the first well and the second well in the second direction.

The first well has a reduced-diameter region of which an opening diameter continuously decreases without increasing at a position closer to the bottom portion than the main surface of the base portion.

Advantageous Effects of Invention

With the cell culture chip according to the present invention, a liquid retained in an opening is able to be sucked by a simple operation procedure while a liquid in a channel remains.

Brief Description of Drawings

[Fig. 1] Fig. 1 is a plan view schematically illustrating a structure of an embodiment of a cell culture chip.
[Fig. 2] Fig. 2 is a sectional view schematically illustrating the structure of the embodiment of the cell culture chip.
[Fig. 3] Fig. 3 is a partially enlarged view of Fig. 2.
[Fig. 4] Fig. 4 is a sectional view schematically illustrating a cell culture chip with a culture solution being filled in.
[Fig. 5A] Fig. 5A is a sectional view schematically illustrating the cell culture chip with the culture solution filled therein being sucked.

[Fig. 5B] Fig. 5B is a sectional view schematically illustrating the cell culture chip with the culture solution filled therein being sucked, and corresponds to a state in which the suction has progressed from Fig. 5A.

[Fig. 5C] Fig. 5C is a sectional view schematically illustrating the cell culture chip with the culture solution filled therein being sucked, and corresponds to a state in which the suction has progressed from Fig. 5B.

[Fig. 6] Fig. 6 is a sectional view schematically illustrating a conventional microchannel chip with a culture solution filled therein being sucked.

[Fig. 7] Fig. 7 is a sectional view schematically illustrating a partial structure of another embodiment of the cell culture chip.

[Fig. 8] Fig. 8 is a plan view schematically illustrating a structure of another embodiment of the cell culture chip.

[Fig. 9] Fig. 9 is a sectional view schematically illustrating a structure of another embodiment of the cell culture chip.

[Fig. 10] Fig. 10 is a sectional view schematically illustrating a structure of another embodiment of the cell culture chip.

[Fig. 11] Fig. 11 is a plan view schematically illustrating a structure of another embodiment of the cell culture chip.

[Fig. 12A] Fig. 12A is a plan view schematically illustrating a structure of another embodiment of the cell culture chip.

[Fig. 12B] Fig. 12B is a plan view schematically illustrating a structure of another embodiment of the cell culture chip.

[Fig. 12C] Fig. 12C is a plan view schematically illustrating a structure of another embodiment of the cell culture chip.

[Fig. 12D] Fig. 12D is a plan view schematically illustrating a structure of another embodiment of the cell culture chip.

[Fig. 13] Fig. 13 is a perspective view schematically illustrating a structure of a conventional microchannel chip.

[Fig. 14] Fig. 14 is a sectional view schematically illustrating the structure of the conventional microchannel chip.

[Fig. 15] Fig. 15 provides views each schematically illustrating a change in the remaining amount of a culture solution when the culture solution is sucked from the conventional microchannel chip.

Description of Embodiments

An embodiment of a cell culture chip according to the present invention will be described with reference to the drawings. It should be noted that the following drawings are merely schematically illustrated. That is, the dimensional ratios on the drawings and the actual dimensional ratios do not necessarily coincide with each other, and the dimensional ratios do not necessarily coincide with each other between the drawings.

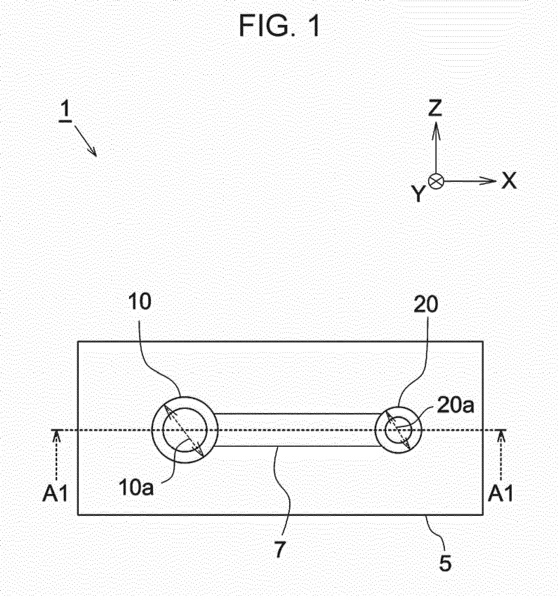

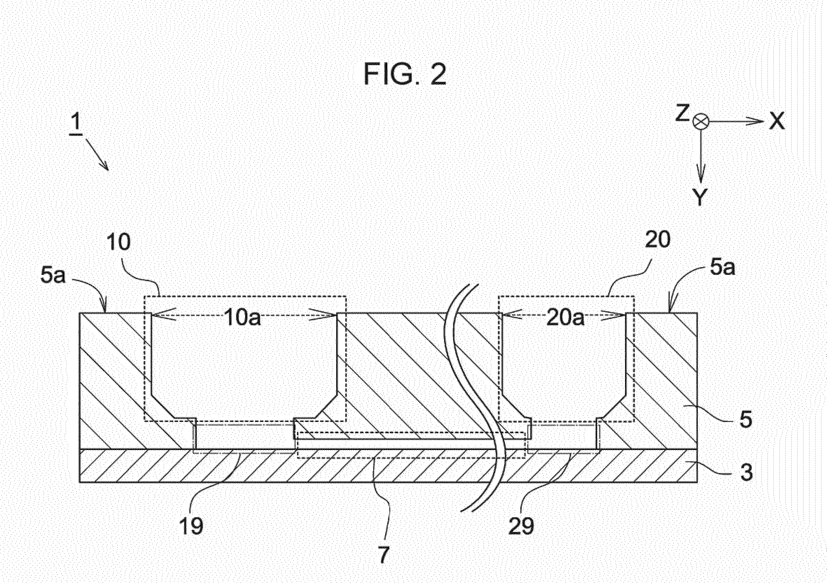

Fig. 1 is a plan view schematically illustrating a structure of an embodiment of a cell culture chip. Fig. 2 is a schematic sectional view when a cell culture chip 1 illustrated in Fig. 1 is cut along line A1-A1 in Fig. 1. In the following description, Fig. 1 corresponds to an XZ plan view when the cell culture chip 1 is viewed in the Y direction, and Fig. 2 corresponds to an XY plan view when the cell culture chip 1 cut along an XY plane is viewed in the Z direction.

The cell culture chip 1 includes a bottom portion 3 and a base portion 5. The base portion 5 includes a first well 10 and a second well 20 that are open in the Y direction toward the bottom portion 3 from a portion of a surface (a main surface 5a) opposite to the bottom portion 3. That is, the first well 10 has an opening area 10a on the main surface 5a side of the base portion 5 and is open in the Y direction toward the bottom portion 3. Similarly, the second well 20 has an opening area 20a on the main surface 5a side of the base portion 5 and is open in the Y direction toward the bottom portion 3. That is, the Y direction corresponds to a "first direction".

The opening area 10a of the first well 10 and the opening area 20a of the second well 20 are disposed at positions separated from each other in a direction parallel to the main surface 5a of the base portion 5. Here, description is given based on the assumption that both are disposed at positions separated from each other in the X direction. In this case, the X direction corresponds to a "second direction". Alternatively, the opening area 10a of the first well 10 and the opening area 20a of the second well 20 may be separated from each other in the Z direction, or may be separated from each other in the X direction and the Z direction. The "second direction" corresponds to a direction extending from the opening area 10a of the first well 10 toward the opening area 20a of the second well 20.

The base portion 5 has a tubular recessed portion at a position on the bottom portion 3 side, and a chamber 7 is formed by a region sandwiched between the recessed portion and the bottom portion 3. In the present embodiment, the chamber 7 constitutes a space in which cells are cultured.

In the present embodiment, one end of the chamber 7 communicates with the first well 10 via a communication well 19, and the other end of the chamber 7 communicates with the second well 20 via a communication well 29. Note that the communication well 19 communicates with the first well 10 in the Y direction, and the bottom portion 3 constitutes a bottom surface of the communication well 19. Similarly, the communication well 29 communicates with the second well 20 in the Y direction, and the bottom portion 3 constitutes a bottom surface of the communication well 29.

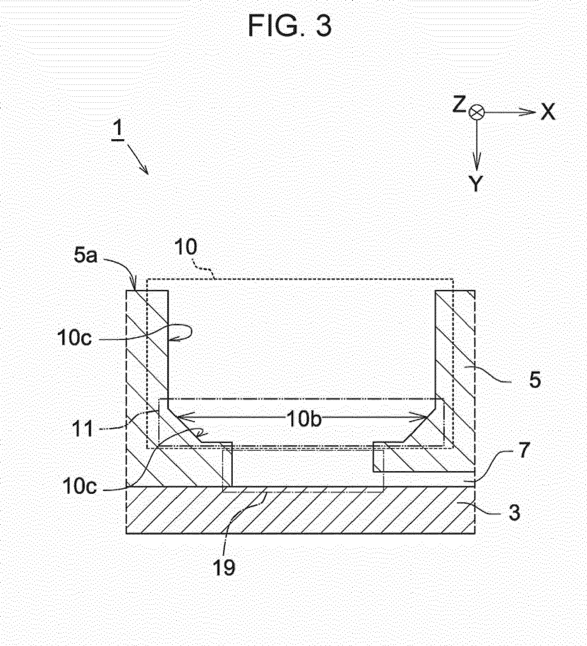

In the present embodiment, the first well 10 and the second well 20 have regions of which opening diameters decrease without increasing toward the bottom portion 3 at positions closer to the bottom portion 3 than the main surface 5a. This point will be described with reference to Fig. 3. Fig. 3 is an enlarged view of the vicinity of the first well 10 of Fig. 2.

As illustrated in Fig. 3, the first well 10 has an opening diameter 10b that decreases without increasing toward the bottom portion 3, that is, as extending in the +Y direction at a position on a side closer to the bottom portion 3 than the main surface 5a of the base portion 5. This region is hereinafter referred to as a "reduced-diameter region 11". In the present embodiment, the opening diameter 10b of the first well 10 is substantially uniform at a position on a side closer to the main surface 5a of the base portion 5 than the reduced-diameter region 11. That is, an inner wall 10c of the first well 10 constitutes an inclined surface in the reduced-diameter region 11.

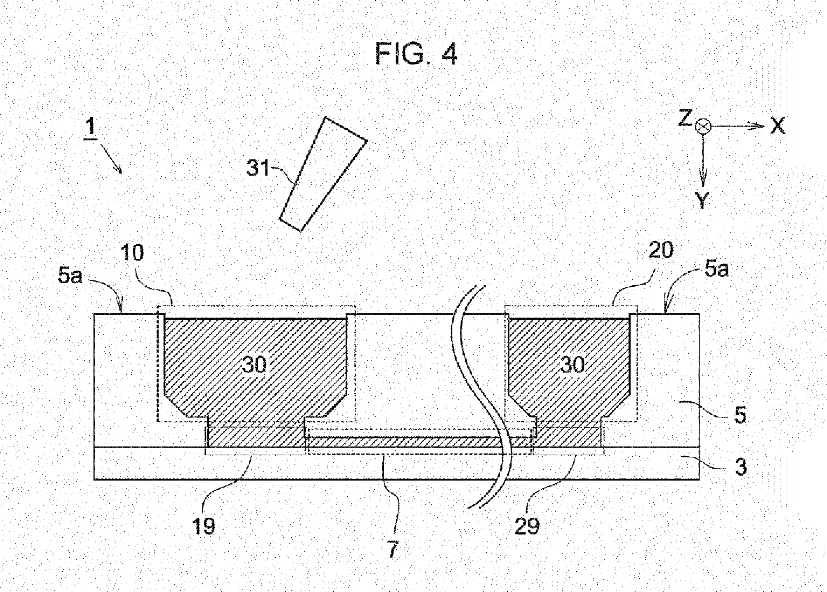

Fig. 4 is a sectional view schematically illustrating the cell culture chip 1 according to the present embodiment with a culture solution 30 filled in. For the convenience of illustration, in Fig. 4, the region where the culture solution 30 exists is hatched, and the bottom portion 3 and the base portion 5 are not hatched. Also in the following drawings, when the region where the culture solution 30 exists is indicated, the culture solution 30 is illustrated in the same manner.

To fill the cell culture chip 1 with the culture solution 30, the culture solution 30 is injected from the first well 10 side or the second well 20 side. For example, when the culture solution 30 is injected from the first well 10 side, the culture solution 30 flows to the second well 20 side via the communication well 19 and the chamber 7. By injecting a predetermined amount or more of the culture solution 30 into the cell culture chip 1, the chamber 7 located between the first well 10 and the second well 20 is filled with the culture solution 30. Thus, cells can be cultured in the chamber 7.

As illustrated in Fig. 4, an example in which the culture solution 30 is extracted using a pipet 31 from a state in which the cell culture chip 1 is filled with the culture solution 30 will be described. Hereinafter, a case where the culture solution 30 is extracted by sucking the culture solution 30 from the first well 10 side using the pipet 31 will be described.

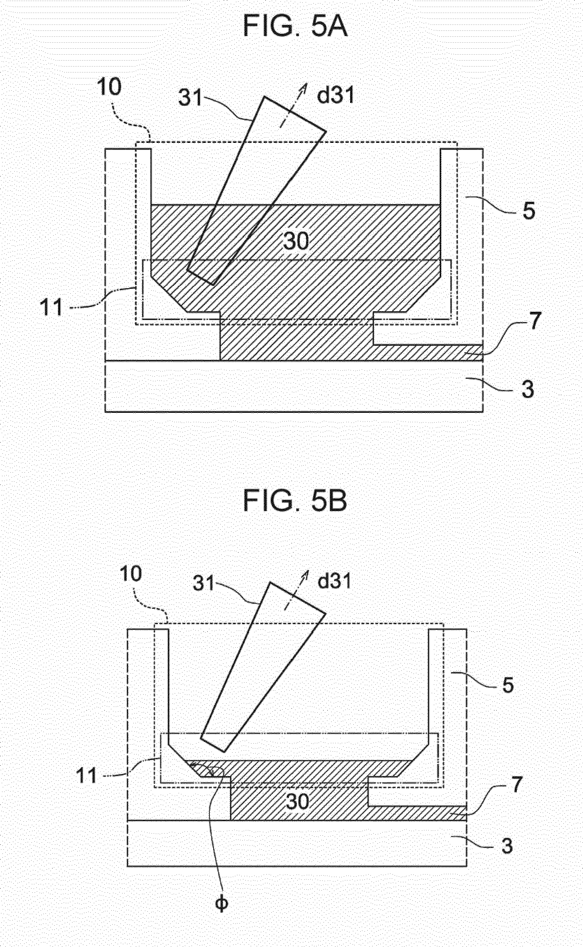

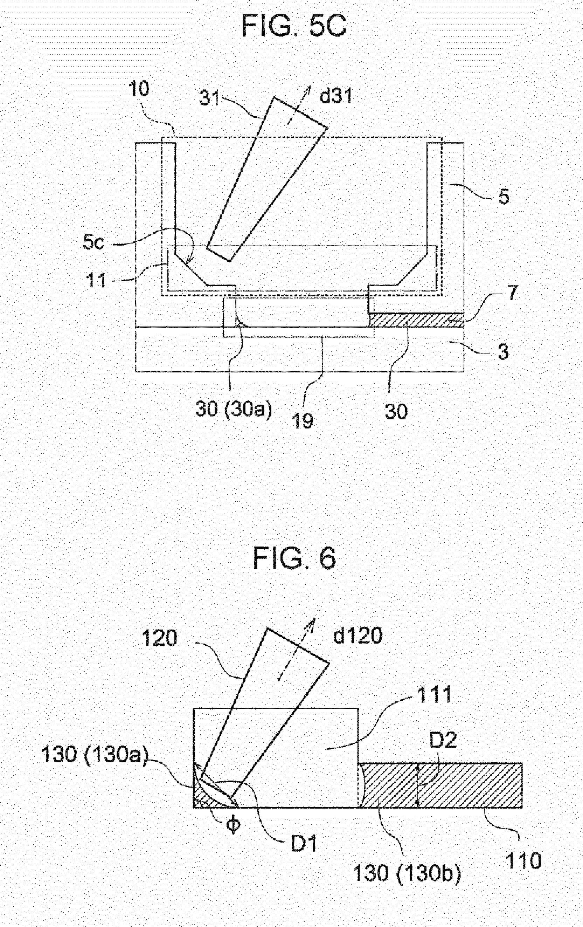

Fig. 5A to Fig. 5C are sectional views schematically illustrating a state in which the culture solution 30 is sucked using the pipet 31. An example in which the suction of the culture solution 30 progresses in the order of Fig. 5A, Fig. 5B, and Fig. 5C is illustrated.

When the tip of the pipet 31 is inserted into the first well 10 and a suction operation is started, the liquid level of the culture solution 30 starts being gradually lowered (see Fig. 5A) and is eventually lowered to locate the liquid level inside of the reduced-diameter region 11 (see Fig. 5B). Then, when the suction operation is further continued, the liquid level of the culture solution 30 is lowered to locate the liquid level inside of the communication well 19 (see Fig. 5C). When the suction operation is continued with a suction force within a range of not less than a suction force capable of completely sucking the culture solution 30 retained in the first well 10 and not more than a predetermined suction force, the liquid level of the culture solution 30 is further lowered, and the bottom portion 3 is eventually exposed (see Fig. 5C). At this time, a liquid pool of the culture solution 30 is formed in a corner portion in the communication well 19 (reference sign 30a). However, even when the suction operation is further continued, the suction of the culture solution 30 does not progress.

That is, according to the configuration of the present embodiment, the culture solution 30 does not flow in from the chamber 7 to the first well 10 side at a time point immediately after the suction of the culture solution 30 retained in the first well 10 is completed. Even when the suction operation from the pipet 31 is continued with the constant suction force, the suction of the culture solution 30 does not progress.

The inventor of the present invention considers the reason why such a phenomenon occurs as follows.

Fig. 6 is a sectional view schematically illustrating a conventional microchannel chip 100 with a culture solution 130 filled therein being sucked, and is substantially the same as Fig. 15(c).

When $\phi$ denotes an angle of a corner portion of an inflow port 111 (hereinafter, referred to as "opening 111" in this case), and D1 denotes a distance between both ends of a portion of a culture solution 130 (130a) where the meniscus of the culture solution 130a retained in the corner portion is in contact with the corner portion, a capillary force P1 generated at the meniscus of the culture solution 130a is expressed by Expression (1) as follows. In the following Expression (1), $\gamma$ indicates a surface tension, and $\theta$ indicates a contact angle of the culture solution 130 (130a).

$$P1 \approx 2 \cdot \gamma \cos(\theta + \phi/2)/(D1/2) \dots (1)$$

In contrast, when D2 is an inner diameter of a groove 110, a capillary force P2 generated at the meniscus of a culture solution 130 (130b) retained in the groove 110 is expressed by the following expression similarly using $\gamma$ and $\theta$. However, in the following Expression (2), since the groove 110 has inner wall surfaces facing each other in parallel and the angle between both ends of a portion of the culture solution 130b where the meniscus of the culture solution 130b is in contact with the inner wall surfaces is 0°, the calculation is performed on the basis of that the component of $\phi$ is 0.

$$P2 \approx 2 \cdot \gamma \cos(\theta)/(D2/2) \dots (2)$$

For example, in a case where the contact angle $\theta$ of the culture solution 130 is 20°, and the inner diameter D2 of the groove 110 is 400 $\mu$m, P1 = P2 is satisfied at the distance D1 being nearly equal to 180 $\mu$m between both ends of the portion where the meniscus of the culture solution 130a is in contact with the corner portion of the opening 111. In other words, at the distance D1 being smaller than 180 $\mu$m, the capillary force P1 generated at the meniscus of the culture solution 130a is larger than the capillary force P2 generated at the meniscus of the culture solution 130b retained in the groove 110, and is P1 > P2.

This means that, in the conventional microchannel chip 100 illustrated in Fig. 6, when the culture solution 130a remaining in the corner portion of the opening 111 is to be sucked to extract the culture solution 130a from the opening 111, the culture solution 130a is not sucked but the culture solution 130b in the groove 110 is sucked. This state corresponds to the fact described above with reference to Fig. 15(d).

In view of this fact, in the structure illustrated in Fig. 3, by making the capillary force of the meniscus of the culture solution 30 retained in the corner portion of the first well 10 smaller than the capillary force of the meniscus of the culture solution 30 in the chamber 7, it is possible to prevent the culture solution 30 from flowing out from the chamber 7 side even when the culture solution 30 in the first well 10 is sucked with a suction force to the extent that the culture solution 30 in the first well 10 can be entirely sucked.

Here, as described above with reference to Fig. 3, the cell culture chip 1 of the present embodiment includes, on the bottom portion 3 side of the first well 10, the reduced-diameter region 11 of which the opening diameter 10b decreases as extending in the +Y direction. The corner angle $\phi$ of the corner portion in the reduced-diameter region 11 is an obtuse angle as illustrated in Fig. 5B and is larger than that of the opening 111 of the conventional microchannel chip illustrated in Fig. 6. Consequently, the value of $\cos(\theta + \phi/2)$ in the above-described Expression (1) decreases. Thus, in the state of Fig. 5B, the value of the capillary force P1 generated at the meniscus of the culture solution 30 retained in the corner portion in the reduced-diameter region 11 is smaller than the value of the capillary force P1 generated at the meniscus of the culture solution 130 (130a) retained in the corner portion of the opening 111 in the conventional structure illustrated in Fig. 6.

Consequently, the capillary force P1 generated at the meniscus of the culture solution 30 retained in the corner portion in the reduced-diameter region 11 becomes smaller than the capillary force P2 of the meniscus of the culture solution 30 in the chamber 7. Accordingly, even when the suction of the culture solution 30 is continued by the pipet 31 from the state illustrated in Fig. 5B, the suction of the culture solution 30 is continued without the culture solution 30 being retained in the corner portion of the first well 10, and the state can progress to the state illustrated in Fig. 5C.

The cell culture chip 1 of the present embodiment includes the communication well 19, and the corner angle of the corner portion of the communication well 19 may be, for example, approximately 90°, similarly to the opening 111 of the conventional microchannel chip 100. In this case, when the suction operation is continued from the state of Fig. 5B, the culture solution 30 (30a) is retained in the corner portion of the communication well 19. However, since at least the culture solution 30 in the first well 10 has been completely removed in a state before the state of Fig. 5C, the suction operation is ended at this time point, and a new culture solution for exchange can be poured from the first well 10 side.

In the case where the contact angle $\theta$ of the culture solution 30 and the height of the chamber 7 are predetermined, the preferable minimum value of the length D of the inclined surface (the length of a chamfered portion) when the inner wall 10c in the reduced-diameter region 11 of the first well 10 is viewed in the Z direction is as provided in Table 1 below. By providing the inner wall 10c in the reduced-diameter region 11 as an inclined surface having a value larger than the value described in the table, the capillary force of the corner portion in the first well 10 can be made smaller than the capillary force of the chamber 7.

[Table 1]

| Length of inclined surface D [$\mu$m] | | Height of chamber 7 [$\mu$m] | | | | | |
|---|---|---|---|---|---|---|---|
| | | 200 | 300 | 400 | 500 | 600 | 900 |
| Contact angle $\theta$ [°] | 5 | 129 | 194 | 258 | 323 | 387 | 516 |
| | 10 | 116 | 175 | 233 | 291 | 349 | 466 |
| | 20 | 90 | 135 | 180 | 225 | 270 | 360 |
| | 40 | 23 | 34 | 46 | 57 | 68 | 91 |

Specific examples of dimensions of the cell culture chip 1 are as follows.

- The height (the length in the Y direction) of the base portion 5 is 1 mm or more and 20 mm or less, and is 3 mm as an example.
- The height (the length in the Y direction) of the bottom portion 3 is 0.1 mm or more and 5 mm or less, and is 1 mm as an example.
- The height (the length in the Y direction) of the chamber 7 is 200 $\mu$m or more and 2000 $\mu$m or less, and is 400 $\mu$m as an example.
- The opening diameter 10b on the opening area 10a side of the first well 10 is 0.5 mm or more and 40 mm or less, and is 2 mm as an example.

- The minimum value of the opening diameter 10b in the reduced-diameter region 11 of the first well 10 is 0.5 mm or more and 40 mm or less, and is 1.75 mm as an example. Also, the length of the inclined surface when the inner wall 10c in the reduced-diameter region 11 is viewed in the Z direction is 20 µm or more and 2000 µm or less, and is 180 µm as an example.
- The opening diameter of the communication well 19 is 0.2 mm or more and 39 mm or less, and is 1.75 mm as an example. Also, the height (the length in the Y direction) of the communication well 19 is 0.2 mm or more and 3 mm or less, and is 0.6 mm as an example.
- The separation distance between the central axis of the first well 10 and the central axis of the second well 20 is 2 mm or more and 40 mm or less, and is 9 mm as an example.
- The opening diameter on the opening area 20a side of the second well 20 is 0.5 mm or more and 40 mm or less, and is 1 mm as an example.
- The minimum value of the opening diameter in the reduced-diameter region of the second well 20 is 0.5 mm or more and 40 mm or less, and is 0.75 mm as an example. Also, the length of the inclined surface when the inner wall 10c in the reduced-diameter region 11 is viewed in the Z direction is 20 µm or more and 2000 µm or less, and is 180 µm as an example.
- The opening diameter of the communication well 29 is 0.2 mm or more and 39 mm or less, and is 0.7 mm as an example. Also, the height (the length in the Y direction) of the communication well 19 is 0.2 mm or more and 2000 mm or less, and is 0.6 mm as an example.

(Modification)

The cell culture chip 1 of the present embodiment can be variously modified. This will be described below.

<1 > The cell culture chip 1 of the above-described present embodiment has the structure in which the second well 20 side also has the reduced-diameter region similar to the reduced diameter region 11 of the first well 10. Thus, even when the culture solution 30 is sucked from the second well 20 side by the pipet 31, the culture solution in the second well 20 can be removed while the culture solution 30 is retained in the chamber 7 for the same reason. However, the present invention does not exclude a structure having a reduced-diameter region only on one well (the first well 10/the second well 20) side.

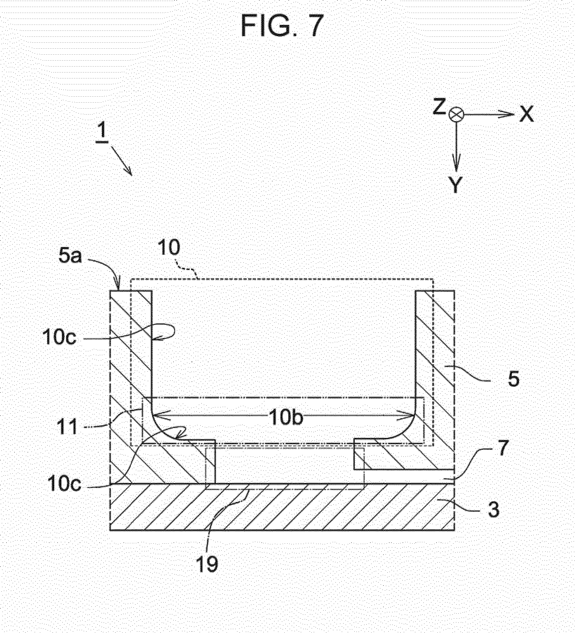

<2> Fig. 3 illustrates the case where the inner wall 10c of the first well 10 in the reduced-diameter region 11 of the cell culture chip 1 is the flat surface. However, from the viewpoint of reducing the capillary force in the corner portion of the first well 10, the inner wall 10c is not necessarily the flat surface, and may be a curved surface. Fig. 7 is a view illustrating a structure in the vicinity of the first well 10 in a case where the inner wall 10c of the first well 10 in the reduced-diameter region 11 is constituted by a curved surface, in a schematic manner like Fig. 3.

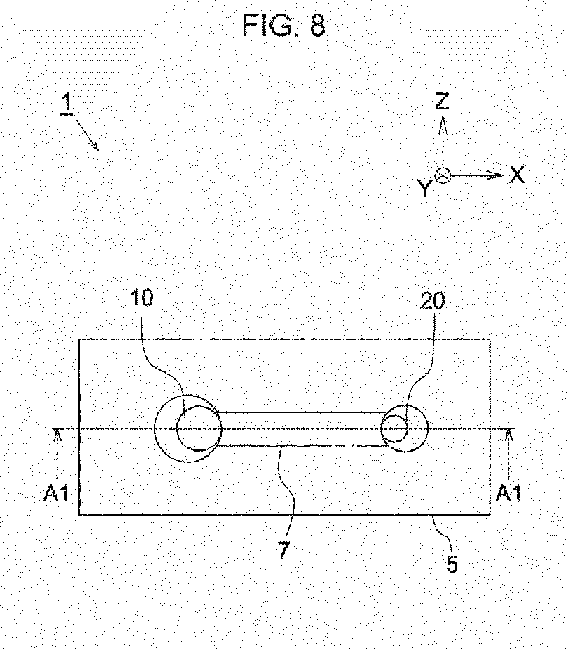

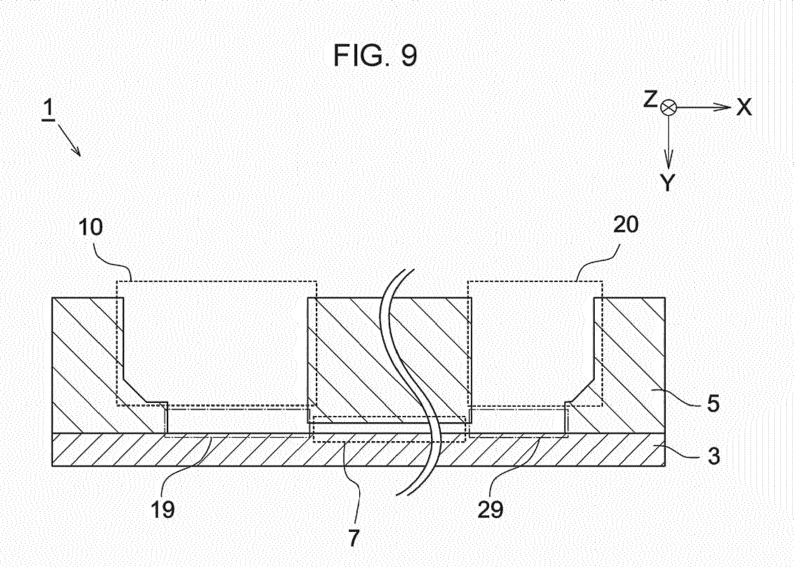

<3> Fig. 2 and Fig. 3 illustrate a case where the first well 10 is of an example in which the opening diameter 10b decreases symmetrically with respect to the central axis in the reduced-diameter region 11. However, the first well 10 may have an eccentric shape such that the central axis in the reduced-diameter region 11 and the central axis on the main surface 5a side of the base portion 5 with respect to the reduced-diameter region 11 are different from each other. Fig. 8 and Fig. 9 illustrate a cell culture chip 1 having such a structure, in a manner similar to Fig. 1 and Fig. 2. In this example, as illustrated in Fig. 7, the inner wall in the reduced-diameter region 11 may be defined only by a curved surface, and the reduced-diameter region 11 may be present only in the first well 10 and a reduced-diameter region may not be provided on the second well 20 side.

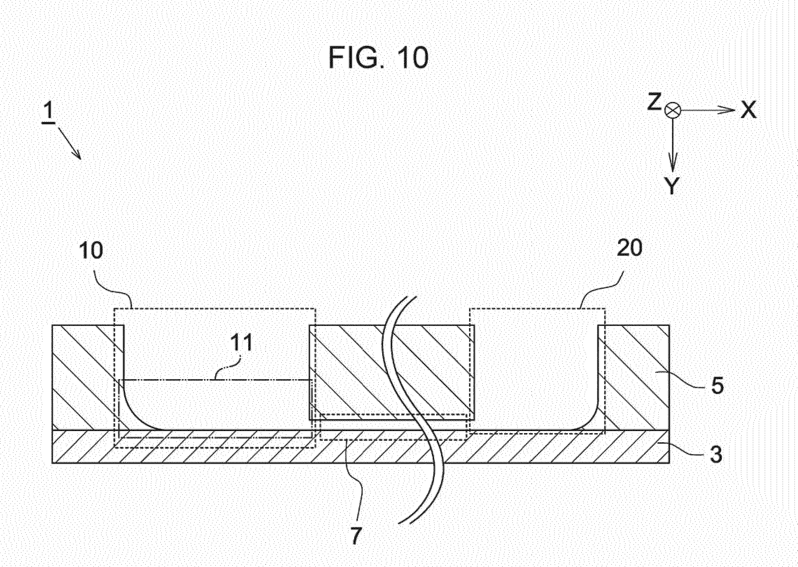

<4> As illustrated in Fig. 10, the cell culture chip 1 may not include the communication well (19/29). Also with such a configuration, since the first well 10 includes the reduced-diameter region, the culture solution 30 in the first well 10 can be removed without substantially sucking the culture solution 30 from the chamber 7 side for the reason described above.
However, as illustrated in Fig. 2, when the cell culture chip 1 includes the communication well (19/29), the thickness (the wall thickness) corresponding to the height of the communication well (19/29) is secured in the base portion 5 located at the position. Thus, from the viewpoint of enabling stable molding at the time of manufacturing the cell culture chip 1, it is preferable to include the communication well (19/29).

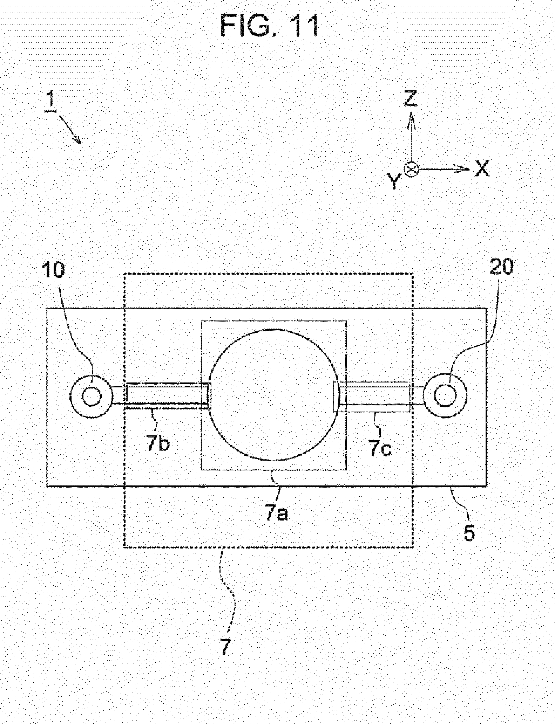

<5> As illustrated in Fig. 11, a chamber 7 included in a cell culture chip 1 may include a culture chamber 7a that substantially constitutes a space in which cells are cultured, and a communication channel (7b/7c) that provides communication between the culture chamber 7a and a corresponding well (10/20) and that has an opening diameter smaller than that of the culture chamber 7a. In this case, it is sufficient that the reduced-diameter region 11 is formed so that the capillary force of the first well 10 is smaller than the capillary force of the communication channel 7b.

Note that, in the structure of the cell culture chip 1 illustrated in Fig. 1 and Fig. 2, as described above, the chamber 7 constitutes a space in which cells are cultured. That is, the chamber 7 corresponds to a culture chamber.

[Other Embodiments]

Other embodiments will be described below.

<1> In the above-described embodiment, the bottom portion 3 and the base portion 5 included in the cell culture chip 1 are described as being provided by separate members, however the bottom portion 3 and the base portion 5 may be formed by integral molding into a single member.

<2> The first well 10 included in the cell culture chip 1 described with reference to Fig. 3 has the substantially uniform opening diameter 10b at a position on a side closer to the main surface 5a of the base portion 5 than the reduced-diameter region 11. However, the first well 10 may have a structure in which the first well 10 has a reduced-diameter region 11 of which an opening diameter 10b decreases over the entire region from the opening area 10a to the bottom portion 3 side.

<3> According to the cell culture chip 1 of each embodiment described above, it has been described that the culture solution 30 filled inside can be extracted from the first well 10 side or the second well 20 side while the culture solution 30 is retained in the chamber 7. However, the substance to be extracted from the cell culture chip 1 while being retained in the chamber 7 is not limited to the culture solution 30, and may be any liquid.

<4> In the above-described embodiment, the case where the pipet 31 is used when the culture solution 30 is sucked from the cell culture chip 1 has been described as an example, however the suction method is not limited to the pipet 31. The cell culture chip 1 of the present invention can employ another typical method of disposing the tip of a suction instrument on one well (for example, the first well 10) side and sucking the culture solution 30 retained in the well.

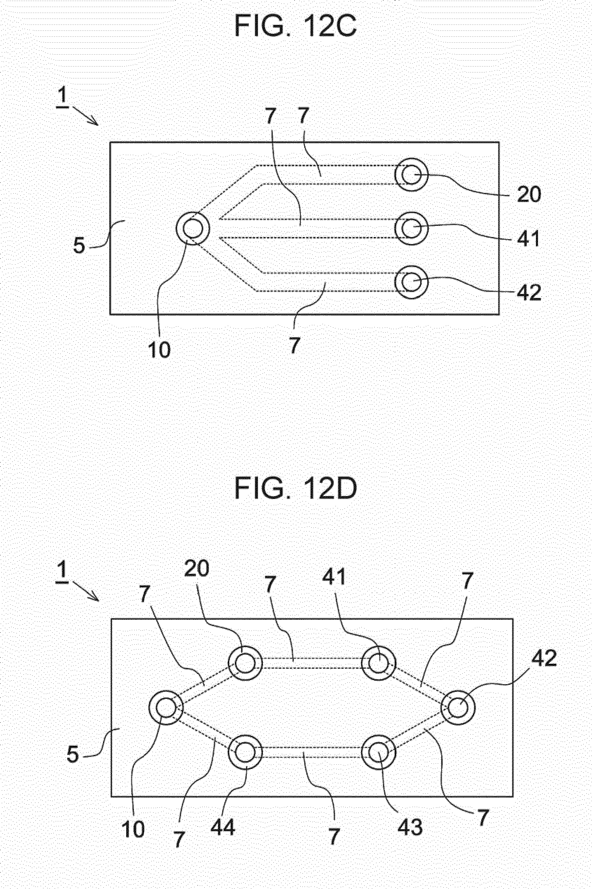

<5> In the above-described embodiment, the cell culture chip 1 in which the pair of wells (10, 20) communicate with each other through the chamber 7 has been described. However, in the cell culture chip 1 of the present invention, the number of wells and the number of chambers are not limited.

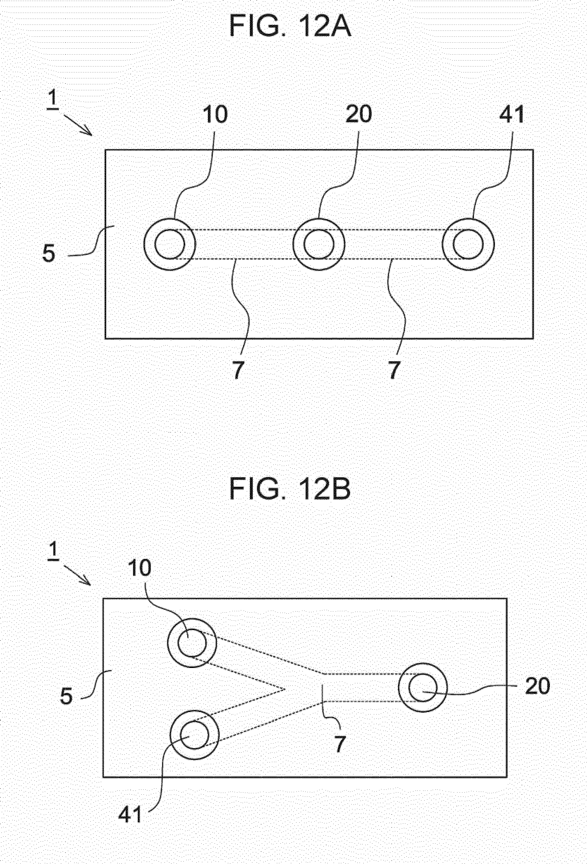

Fig. 12A to Fig. 12D are plan views schematically illustrating a cell culture chip 1 according to another embodiment in a manner similar to Fig. 1.
In the cell culture chip 1 illustrated in Fig. 12A, three wells (10, 20, 41) are formed in series, and chambers (7, 7) are formed to provide communication between the wells. In the cell culture chip 1 illustrated in Fig. 12B, three wells (10, 20, 41) are formed. However, unlike the cell culture chip 1 illustrated in Fig. 12A, a chamber 7 that provides communication between the well 10 and the well 20 and a chamber 7 that provides communication between the well 10 and the well 41 communicate with each other.
A cell culture chip 1 illustrated in Fig. 12C includes four wells (10, 20, 41, 42), and a chamber 7 that provides communication between the well 10 and the well 20, a chamber 7 that provides communication between the well 10 and the well 41, and a chamber 7 that provides communication between the well 10 and the well 42 are independently formed.
A cell culture chip 1 illustrated in Fig. 12D includes six wells (10, 20, 41, 42, 43, 44), and a chamber 7 is formed to provide communication between the adjacent wells. Note that all the wells (10, 20, 41, 42, 43, 44) communicate with one another in series in a ring shape through each chamber 7.
The cell culture chip 1 illustrated in each of Fig. 12A to Fig. 12D also has a shape such that the capillary forces of the wells (10, 20, 41, 42, 43, 44) are smaller than the capillary forces of the chambers 7. Since an example of a more specific structure is common to that of the above-described embodiment, the description thereof will be omitted.

<6> In the cell culture chip 1 described with reference to Fig. 2, the communication wells (19, 29) are described as providing communication between the respective wells (10, 20) and the chamber 7 in the Y direction. However, the communication wells (19, 29) may be formed to provide communication between the respective wells (10, 20) and the chamber 7 also in a direction (for example, the X direction) parallel to the main surface 5a of the base portion 5. That is, ends of the chamber 7 may not be disposed directly below the wells (10, 20), and the communication wells (19, 29) may extend also in the X direction to provide communication between the wells (10, 20) and the chamber 7.

Reference Signs List

| 1: | cell culture chip |
| 3: | bottom portion |
| 5: | base portion |
| 5a: | main surface of base portion |
| 7: | chamber |
| 7a: | culture chamber |
| 7b, 7c: | communication channel |
| 10: | first well |
| 10a: | opening area of first well |
| 10b: | opening diameter of first well |
| 10c: | inner wall of first well |
| 11: | reduced-diameter region |
| 19: | communication well |
| 20: | second well |
| 20a: | opening area of second well |
| 29: | communication well |
| 30, 30a: | culture solution |
| 31: | pipet |
| 41,42,43,44: | well |
| 100: | conventional microchannel chip |
| 101: | base |
| 102: | resin film |
| 110: | groove |
| 111: | inflow port |
| 112: | outflow port |
| 120: | pipet |
| 130, 130a: | culture solution |

**Claims**

1. A cell culture chip comprising:

a bottom portion;
a base portion formed on an upper surface of the bottom portion;
a first well provided by opening the base portion in a first direction extending from a portion of a main surface of the base portion toward the bottom portion, the main surface being a surface opposite to the bottom portion;
a second well provided by opening the base portion in the first direction at a position separated from the first well in a second direction parallel to the main surface; and
a tubular chamber that is defined by a region sandwiched between the bottom portion and the base portion and that provides communication between the first well and the second well in the second direction,
wherein the first well has a shape such that a capillary force of the first well is smaller than a capillary force of the chamber.

2. The cell culture chip according to Claim 1, wherein the first well has a reduced-diameter region of which an opening diameter continuously decreases without increasing toward the bottom portion at a position closer to the bottom portion than the main surface of the base portion.

3. The cell culture chip according to Claim 2, wherein the first well has an inner wall defined by the base portion, and wherein the inner wall includes a curved surface or a flat surface non-parallel to the main surface in the reduced-diameter region of the first well.

4. The cell culture chip according to Claim 2 or 3, comprising:

a communication well that is formed continuously with the first well in the first direction and that provides communication between the reduced-diameter region of the first well and the chamber in the first direction, wherein the communication well has a bottom surface defined by the bottom portion and has an opening diameter smaller than the opening diameter of the reduced-diameter region of the first well.

5. The cell culture chip according to Claim 2 or 3, wherein a bottom surface of the first well is defined by the bottom portion.

6. The cell culture chip according to any one of Claims 1 to 5, wherein the second well has a shape such that a capillary force of the second well is smaller than a capillary force of the chamber.

7. The cell culture chip according to Claim 6, wherein the second well has a reduced-diameter region of which an opening diameter continuously decreases without increasing toward the bottom portion at a position closer to the bottom portion than the main surface of the base portion.

8. A cell culture chip comprising:

a bottom portion;
a base portion formed on an upper surface of the bottom portion;
a first well provided by opening the base portion in a first direction extending from a portion of a main surface of the base portion toward the bottom portion, the main surface being a surface opposite to the bottom portion;
a second well provided by opening the base portion in the first direction at a position separated from the first well in a second direction parallel to the main surface; and
a tubular chamber that is defined by a region sandwiched between the bottom portion and the base portion and that provides communication between the first well and the second well in the second direction,
wherein the first well has a reduced-diameter region of which an opening diameter continuously decreases without increasing at a position closer to the bottom portion than the main surface of the base portion.

9. The cell culture chip according to Claim 8, wherein the first well has an inner wall defined by the base portion, and wherein the inner wall includes a curved surface or a flat surface non-parallel to the main surface in the reduced-diameter region of the first well.

10. The cell culture chip according to Claim 8 or 9, wherein the second well has a reduced-diameter region in which an opening diameter continuously decreases without increasing at a position closer to the bottom portion than the main surface of the base portion.

# FIG. 1

## FIG. 2

# FIG. 3

# FIG. 4

## FIG. 5A

## FIG. 5B

## FIG. 5C

## FIG. 6

FIG. 7

# FIG. 8

EP 3 858 974 A1

FIG. 9

EP 3 858 974 A1

# FIG. 10

# FIG. 11

1

Z

Y⊗ →X

10    20

7b    7c

7a

5

7

## FIG. 12A

## FIG. 12B

## FIG. 12C

## FIG. 12D

# FIG. 13

d112

112

111

d111

101

102

110

100

# FIG. 14

100

111

112

101

102

110

FIG. 15

(a)

(b)

(c)

(d)

## INTERNATIONAL SEARCH REPORT

| International application No. |
|---|
| PCT/JP2019/035653 |

**A. CLASSIFICATION OF SUBJECT MATTER**
Int.Cl. C12M3/00(2006.01)i, B81B1/00(2006.01)i, C12M1/00(2006.01)i

According to International Patent Classification (IPC) or to both national classification and IPC

**B. FIELDS SEARCHED**

Minimum documentation searched (classification system followed by classification symbols)
Int.Cl. C12M3/00, B81B1/00, C12M1/00

Documentation searched other than minimum documentation to the extent that such documents are included in the fields searched
Published examined utility model applications of Japan    1922–1996
Published unexamined utility model applications of Japan  1971–2019
Registered utility model specifications of Japan          1996–2019
Published registered utility model applications of Japan  1994–2019

Electronic data base consulted during the international search (name of data base and, where practicable, search terms used)
JSTPlus/JMEDPlus/JST7580(JDreamIII), CAplus/MEDLINE/EMBASE/BIOSIS(STN),
WPIDS/WPIX(STN)

**C. DOCUMENTS CONSIDERED TO BE RELEVANT**

| Category* | Citation of document, with indication, where appropriate, of the relevant passages | Relevant to claim No. |
|---|---|---|
| X | JP 2005-103423 A (FUJI KAGAKU KK) 21 April 2005, paragraphs [0001], [0002], [0036], [0037], fig. 1-4 (Family: none) | 1-10 |
| Y | | 1-10 |
| Y | JP 2011-128019 A (HITACHI MAXELL LTD.) 30 June 2011, claims, paragraphs [0004], [0025], fig. 3, 6 (Family: none) | 1-10 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 18.11.2019 | 26.11.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2019/035653

C (Continuation). DOCUMENTS CONSIDERED TO BE RELEVANT

| Category* | Citation of document, with indication, where appropriate, of the relevant passages | Relevant to claim No. |
|---|---|---|
| A | JP 2018-47614 A (SUMITOMO BAKELITE CO., LTD.) 29 March 2018, entire text, all drawings (Family: none) | 1-10 |
| A | JP 2012-211870 A (SHIMADZU CORPORATION) 01 November 2012, entire text, all drawings (Family: none) | 1-10 |
| A | JP 2016-103982 A (TOKYO ELECTRON LTD.) 09 June 2016, entire text, all drawings & WO 2014/142161 A1 | 1-10 |
| P, X | JP 2019-32233 A (ENPLAS CORPORATION) 28 February 2019, claims, paragraphs [0022], [0023], [0028]-[0033], fig. 1, 2 & WO 2019/031236 A1 | 1-10 |

Form PCT/ISA/210 (continuation of second sheet) (January 2015)

**EP 3 858 974 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018047614 A